(19) Europäisches Patentamt
European Patent Office
Office européen des brevets

(11) Numéro de publication: **0 084 743**
**B1**

(12) # FASCICULE DE BREVET EUROPEEN

(45) Date de publication du fascicule du brevet: **21.11.84**

(51) Int. Cl.[3]: **C 07 C 147/06**, C 07 C 149/34, C 07 C 148/00

(21) Numéro de dépôt: **82401216.5**

(22) Date de dépôt: **30.06.82**

(54) **Procédé de préparation de alpha,alpha-difluoroalkoxy ou alpha,alpha-difluoroalkylthiophenyl sulfones.**

(30) Priorité: **21.01.82 FR 8200878**

(43) Date de publication de la demande: **03.08.83 Bulletin 83/31**

(45) Mention de la délivrance du brevet: **21.11.84 Bulletin 84/47**

(84) Etats contractants désignés: **AT BE CH DE FR GB IT LI LU NL SE**

(56) Documents cités:
**DE - A - 1 645 153**
**FR - A - 2 053 336**

(73) Titulaire: **RHONE-POULENC SPECIALITES CHIMIQUES, "Les Miroirs" 18, Avenue d'Alsace, F-92400 Courbevoie (FR)**

(72) Inventeur: **Desbois, Michel, 526, Chemin du Bois, F-69140-Rillieux (FR)**

(74) Mandataire: **Cazes, Jean-Marie et al, RHONE-POULENC RECHERCHES Service Brevets Chimie et Polymères 25, quai Paul Doumer, F-92408 Courbevoie Cedex (FR)**

**EP 0 084 743 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention a pour objet un procédé de préparation de $\alpha,\alpha$-difluoroalkoxy ou $\alpha,\alpha$-difluoroalkylthiophénylsulfones.

On connait dans l'art antérieur des documents décrivant la préparation de composés de ce type.

C'est ainsi que la référence Chemical abstracts 61.8217 décrit la préparation de p-$F_3COC_6H_4SO_2CF_3$. Selon le procédé décrit, la paratrifluorométhoxyphénylaniline, elle-même obtenue par nitration du parafluorométhoxybenzène est soumise à une diazotation en milieu HCl aqueux, le produit résultant est d'abord traité par $EtOCS_2K$ puis à la potasse, puis par le diméthylsulfate pour donner le paratrifluorométhoxythioanisole. Ce dernier composé est chloré pour donner le paratrifluorométhoxytrichlorométhylthiobenzène. Ensuite, ce composé est traite à $SbF_3$ pour donner le paratrifluorométhoxytrifluorométhylthiobenzène qui est converti en produit recherché par oxydation chromique dans l'acide acétique.

On connait également la référence Chemical Abstracts 87.134 226 h qui décrit la préparation de m-$CF_3S-C_6H_4-SO_2-CF_3$ par trifluorométhylation avec $CF_3I$ du thiol correspondant dans l'ammoniaque liquide sous irradiation ultraviolette.

A la lecture de ces deux documents, l'homme de l'art constate que ces procédés sont très difficilement transposables à l'échelle industrielle, du fait des nombreuses étapes nécessaires, de la difficulté de mise en œuvre de chacune de ces étapes ou encore de la non-accessibilité industrielle de certains réactifs impliqués dans ces procédés.

Il faut souligner, de plus, les mauvais rendements obtenus.

Les trifluorométhylthiophénylsulfones sont préparées dans l'art antérieur par réaction de Grignard sur le benzonitrile correspondant (voir par exemple le brevet des Etats-Unis d'Amérique 3732307). La réaction de Grignard limite, bien évidemment, l'intérêt industriel de ce type de procédé.

On connait encore dans l'art antérieur (Olah »Friedel-Crafts and Related Reactions — III — Part II — 1964 — Interscience Publishers« p. 1319 et suivantes) des réactions de sulfonylation directe, c'est-à-dire la réaction d'un acide sulfonique, d'un dérivé ou d'un précurseur de ce dernier sur des dérivés benzéniques en présence de chlorure d'aluminium notamment.

Les expériences de la demanderesse ont montré que ces procédés ne donnent aucun résultat lorsque le composé benzénique porte un substituant polyhalogénoalkoxy ou polyhalogénoalkylthio et, notamment, $OCF_3$ iz $SCF_3$ (ces derniers sont même dégradés).

La demanderesse a maintenant découvert les conditions opératoires permettant de mettre en œuvre la réaction de sulfonylation sur les substrats aromatiques ayant un substituant polyhalogénoalkoxy ou polyhalogénoalkylthio, ce qui n'avait pu être obtenu dans l'art antérieur.

La présente invention concerne un procédé de préparation de $\alpha,\alpha$-difluoroalkoxy ou $\alpha,\alpha$-difluoroalkylthiophénylsulfones caractérisé en ce que l'on fait réagir un polyhalogénoalkoxybenzène ou un polyhalogénoalkylthiobenzène avec un acide sulfonique, un dérivé ou un précurseur de cet acide, en présence de trifluorure de bore en quantité telle que la pression absolue de trifluorure de bore dans l'enceinte réactionnelle soit supérieure à 1 bar et en présence d'acide fluorhydrique comme solvant.

Le procédé selon l'invention présente un caractère d'autant plus surprenant qu'il est précisé dans l'art antérieur (OLAH précité p. 1338) que le trifluorure de bore $BF_3$ est inactif lorsqu'il est employé comme catalyseur dans les réactions de sulfonylation.

On entend au sens de la présente invention par polyhalogénoalkoxybenzène ou polyhalogénoalkylthiobenzène, d'une part, ces produits proprement dits et, d'autre part, leurs homologues correspondant à la présence d'un ou plusieurs radicaux substituant le noyau benzénique.

Plus particulièrement encore, les polyhalogénoalkoxybenzènes ou polyhalogénoalkylthiobenzènes concernés par la présente invention ont pour formule générale:

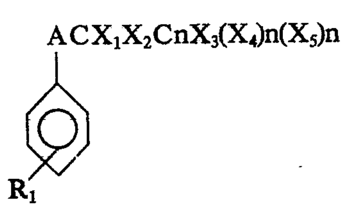

(I)

dans laquelle $X_1$ et $X_2$ identiques ou différents représentent Cl, Br, I ou F, $X_3$, $X_4$ et $X_5$ identiques ou différents représentent H, Cl, Br, I ou F, n est un nombre entier supérieur ou égal à O et inférieur ou égal à 5 ($0 \leq n \leq 5$), A représente O ou S et $R_1$ représente au moins un élément choisi parmi le groupe comprenant l'hydrogène et les radicaux alkyle et alkoxy ayant de 1 à 6 atomes de carbone environ, les radicaux phényle et phénoxy substitués par au moins un groupement plus désactivant que le groupement $ACX_1X_2CnX_3(X_4)n(X_5)n$, OH, Cl, Br, I et F.

Les radicaux $R_1$ phényle et phénoxy doivent être substitués par des groupements plus désactivants que le groupement $ACX_1X_2Cn(X_3)$-$(X_4)n$ $(X_5)n$ pour que la réaction d'acylation intervienne sur le noyau benzénique porteur du groupement $ACX_1X_2CnX_3(X_4)n(X_5)n$. Dans le cas contraire, la réaction d'acylation se ferait sur le radical phényle ou phénoxy. On peut citer comme exemples de groupements plus

désactivants que le groupement $ACX_1X_2CnX_3(X_4)n(X_5)n$ les groupements COOH, CN, $NO_2$, $CX_1X_2X_3$ et cétones.

Les composés de formule I dans laquelle n = O ou I et $X_1$ $X_2$ et $X_3$ sont identiques sont encore plus particulièrement concernés par la présente invention. On préfère mettre en œuvre parmi ceux-ci les composés dans lesquels $X_1$ $X_2$ et $X_3$ représentent le fluor.

On peut citer comme exemples de composés de formule I, les composés suivants: le trifluorométhoxybenzène, le trifluorométhylthiobenzène, l'o-, m- et p-chlorotrifluorométhoxybenzène, l'o-, m- et p-chlorotrifluorométhylthiobenzène, l'o-, m- et p-bromotrifluorométhylthiobenzène, l'o-, m- et p-bromotrifluorométhoxybenzène, l'o-, m- et p-méthyltrifluorométhoxybenzène, l'o-, m- et p-méthoxytrifluorométhoxybenzène, l'o-, m- et p-methoxytrifluorméthylthiobenzene, l'o-, m- et p-hydroxytrifluorométhoxybenzène, l'o-, m- et p-hydroxytrifluorométhylthiobenzène, le trifluorométhyl-4 trifluorométhoxy-4' biphényle, le nitro-3 trifluorométhoxy-4' diphényle oxyde (on peut également citer les homologues chlorés, bromés et iodés des composés ci-dessus) le difluorobromométhoxybenzène, le difluorobromométhylthiobenzène, le dichlorofluorométhoxybenzène, le dichlorofluorométhylthiobenzène, le difluorochlorométhoxybenzène, le difluorochlorométhylthiobenzène, $\alpha,\alpha,\beta,\beta,\beta$-pentachloroéthoxybenzène; $\alpha,\alpha,\beta,\beta,\beta$-pentachloroéthylthiobenzène; difluorométhoxybenzène; difluorométhylthiobenzène; $\alpha,\alpha,\beta,\beta$-tétrafluoroéthoxybenzène; $\alpha,\alpha,\beta,\beta$-tetrafluoroéthylthiobenzène; $\alpha,\alpha,\beta,\beta$-tétrafluoro $\beta$-bromoéthoxybenzène; $\alpha,\alpha,\beta,\beta$-tétrafluoro $\beta$-bromoéthylthiobenzène; $\alpha,\alpha$-difluoro-$\beta,\beta,\beta$-trichloroéthoxybenzène.

On entend au sens de la présente invention par acide sulfonique le précurseur ou le dérivé de cet acide, tous les réactifs de sulfonylation bien connus dans l'art antérieur.

Selon un mode de réalisation particulier de la présente invention l'acide sulfonique, le précurseur ou le dérivé de cet acide répond à la formule générale:

$$R_2-SO_2X_6 \qquad (II)$$

dans laquelle:

$R_2$ représente un radical aliphatique ou aromatique
$X_6$ représente un halogène, OH, $OR_3$, $NH_2$, $NHR_4$, $NR_5R_6$ où $R_3$, $R_4$, $R_5$ et $R_6$ sont un radical aromatique ou aliphatique.

L'invention est particulièrement bien adaptée à la mise en œuvre d'un composé II dans laquelle $R_2$ représente un radical alkyle, phényle, alkylphényle, phénylalkyle ou phényle comportant au moins un substituant comme par exemple un halogène, $NO_2$, CN, $NH_2$, COOH.

On peut citer comme exemples de tels composés: chlorure de paratoluènesulfonyle, chlorure de benzène sulfonyle, acide paratoluène sulfonique, acide benzènesulfonique, chlorure de méthanesulfonyle, chlorure d'éthanesulfonyle, chlorure d'orthotoluènesulfonyle, chlorure de 2,4 diméthylbenzènesulfonyle, chlorure de métanitrobenzènesulfonyle, chlorure de méthoxy-2 benzène sulfonyle, chlorure de parachlorobenzènesulfonyle, chlorure de nitro-3 chloro-4 benzène sulfonyle, fluorure de parahydroxybenzènesulfonyle, le chlorure de parabenzylbenzènesulfonyle.

Le procédé selon l'invention est mis en œuvre de préference en utilisant une quantité d'acide fluorhydrique telle que le rapport molaire de l'acide fluorhydrique au composé de formule I est compris entre 5 et 50. Plus particulièrement, ce rapport est compris entre 10 et 30.

L'acide fluorhydrique mis en œuvre est de préference anhydre. La mise en œuvre d'acide fluorhydrique aqueux entrainerait une consommation inutile de trifluorure de base sous forme HF, $BF_3$, $H_2O$ $(H_3O^+BF_4^-)$.

Les composés de formule I et II sont utilisés en quantité sensiblement équimolaire. Un léger excès du premier peut cependant être souhaitable.

On préfère plus particulièrement utiliser une quantité de trifluorure de bore telle que la pression absolue de $BF_3$ dans l'enceinte réactionnelle soit comprise entre 6 et 20 bars. Une pression supérieure à 20 bars n'est pas exlue de l'invention mais n'apporte pas d'avantages particuliers. L'homme de l'art adaptera donc la pression à l'économie du procédé.

Le procédé selon l'invention est de préference mis en œuvre à une température comprise entre −20° C et 150° C.

Les temps de réaction sont généralement compris entre quelques minutes et plusieurs heures.

Les $\alpha,\alpha$-difluoroalkoxy ou $\alpha,\alpha$difluoroalkylthiophénylsulfones obtenues selon le procédé selon l'invention ont pour formule générale:

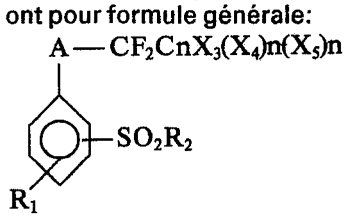

où A, $R_1$ $R_2$ $X_3$ $X_4$ et $X_5$ ont la signification précédente.

Lors de la réaction qui s'effectue en milieu HF, quand dans la formule I n = 0 et $X_3$ est un halogène le composé obtenu comportera le substituant $-A-CF_3$ (les groupements $ACCl_3$, $ACBr_3$, $ACI_3$, $ACF_2Br$, $ACCl_2F$, $ACF_2Cl$, etc. . . se transforment en $ACF_3$).

Quand n = 0 et $X_3$ est H le composé obtenu comportera le substituant $-ACF_2H$. Quand n est supérieur à 0 seuls les substituants $X_1$ et $X_2$ sont échangés par des atomes de fluor sauf s'ils sont initialement des fluor.

La position du groupement $SO_2R_2$ par rapport aux groupements $ACF_2CnX_3[X_4)n(X_5)n$ et $R_1$ obéit aux règles de substitution bien connues du chimiste organicien.

Les sulfones obtenues selon le procédé de l'invention sont utiles notamment comme intermédiaires de synthèse de composés ayant une activité pharmaceutique ou phytosanitaire.

On peut citer comme exemples de composés pouvant être préparés par le procédé selon l'invention:

trifluorométhoxy-4 diphénylsulfone, trifluorométhoxy-4 méthyl-4' diphénylsulfone, trifluorométhylthio-4 méthyl-4' diphénylsulfone, nitro-4 trifluorométhoxy-4' diphénylsulfone, nitro-4 trifluorométhylthio-4' diphénylsulfone, trifluorométhoxy-4 chloro-4' diphénylsulfone, trifluorométhoxy-4 méthyl-2' diphénylsulfone, trifluorométhoxy-4 chloro-2 diphénylsulfone, trifluorométhylthio-4 méthyl-2' diphénylsulfone, trifluorométhoxy-4 phényl-méthylsulfone, trifluorométhylthio-4 phénylméthylsulfone, trifluorométhoxy-4 phényléthylsulfone, trifluorométhylthio-4 diméthyl-2',4' diphénylsulfone, nitro-3 trifluorométhylthio-4' diphénylsulfone, trifluorométhoxy-3 chloro-6 méthyl-4' diphénylsulfone; trifluorométhoxy-2 chloro-5 méthyl-4' diphénylsulfone; $\alpha,\alpha,\beta,\beta$-tétrafluoroéthylthio-4 méthyl-4' diphénylsulfone; ($\alpha,\alpha$-difluoro $\beta,\beta,\beta$-trichloroéthoxy)-4 méthyl-4' diphénylsulfone; $\alpha,\alpha$-difluorométhoxy-4 méthyl-4' diphényl sulfone.

L'invention va être maintenant plus complètement décrite à l'aide des exemples qui vont suivre. Ces derniers ne sauraient être considérés comme limitant de façon quelconque l'invention.

## Exemple 1

Dans un réacteur inox de 250 ml muni d'un système d'agitation magnétique, on introduit aux environs de 0°C, 100 ml d'HF anhydre, 38,1 g (0,2 mole) de chlorure de p-toluène sulfonyle et 32,4 g (0,2 mole) de trifluorométhoxybenzène. On ferme le réacteur puis on introduit du trifluorure de bore ($BF_3$) gazeux jusqu'à la pression constante de 10 bars. On laisse alors réagir sous agitation pendant 20 heures à la température ambiante. Après réaction, on décomprime le réacteur jusqu'à pression atmosphérique puis on coule le mélange réactionnel sur 200 g de glace pilée. On extrait alors le mélange hétérogène ainsi obtenu par trois fois 200 $cm^3$ de chlorure de méthylène. Les phases organiques sont lavées par trois fois 200 $cm^3$ d'eau, l fois 200 $cm^3$ d'une solution aqueuse à 3% de potasse et 2 fois 200 $cm^3$ d'eau. La phase organique ainsi traitée est séchée sur sulfate de magnésium et le solvant est éliminé par distillation sous pression réduite. On recueille ainsi 59 g (rendement: 94%) de trifluorométhoxy-4 méthyl-4' diphényl sulfone de pureté 99,4%. Point de fusion: 75,5—76°C (MeOH).

## Exemple 2

On procède comme à l'exemple I avec les composés et conditions suivantes:

| | |
|---|---|
| — Acide fluorhydrique anhydre | 100 g |
| — Trifluorométhoxybenzène | 32,4 g 0,2 mole |
| — Chlorure de benzène sulfonyle | 35,2 g 0,2 mole |
| — Trifluorure de bore | 10 bars à 20°C |
| — Température | 20°C |
| — Durée | 3 heures |

On recueille 46 g (rendement: 76%) de trifluorométhoxy-4 diphénylsulfone à 98,5% de pureté. Point de fusion: 59—60°C (MeOH).

Exemple 3

On procède comme à l'exemple I avec les composés et conditions suivantes:

- Acide fluorhydrique anhydre — 100 g
- Trifluorométhoxybenzène — 32,4 g 0,2 mole
- Acide p-toluène sulfonique — 34,4 g 0,2 mole
- Trifluorure de bore — 10 bars à 20°C
- Température — 30°C
- Durée — 4 heures

On recueille 45,6 g (rendement: 72%) de trifluorométhoxy-4 méthyl-4' diphénylsulfone à 99% de pureté. Point de fusion: 75,5—76°C (MeOH).

Exemple 4

On procède comme à l'exemple I avec les composés et conditions suivantes:

- Acide fluorhydrique anhydre — 100 g
- Trifluorométhoxybenzène — 32,4 g 0,2 mole
- Chlorure de benzène sulfonyle — 22,9 g 0,2 mole
- Trifluorure de bore — 10 bars à 20°C
- Température — 80°C
- Durée — 5 heures

On recueille 13 g d'un mélange comportant 60% de trifluorométhoxy-4 phényl, méthyl sulfone.

Exemple 5

On procède comme à l'exemple I avec les composés et conditions suivantes:

- Acide fluorhydrique anhydre — 100 g
- Trifluorométhoxybenzène — 17,8 g 0,1 mole
- Chlorure de benzène sulfonyle — 17,6 g 0,1 mole
- Trifluorure de bore — 10 bars à 20°C
- Température — 50°C
- Durée — 20 heures

On recueille 27 g (rendement: 85%) de trifluorométhylthio-4 diphénylsulfone à 70% de pureté.

Exemple 6

On procède comme à l'exemple I avec les composés et conditions suivantes:

- Acide fluorhydrique anhydre — 100 g
- Trifluorométhoxybenzène — 42,3 g 0,2 mole
- Chlorure de benzène sulfonyle — 38,1 g 0,2 mole
- Trifluorure de bore — 10 bars à 20°C
- Température — 50°C
- Durée — 6 heures

On recueille 52 g (rendement: 82,8%) de trifluorométhoxy-4 méthyl-4' diphénylsulfone à 94% de pureté.

On note, au cours de la réaction, une augmentation de pression due au départ d'acide chlorhydrique provenant de l'échange Cl—F.

Exemple 7

On procède comme à l'exemple I avec les composés et conditions suivantes:

| | |
|---|---|
| — Acide fluorhydrique anhydre | 100 g |
| — Difluorobrométhoxybenzène | 44,6 g 0,2 mole |
| — Chlorure de p-toluène sulfonyle | 38,1 g 0,2 mole |
| — Trifluorure de bore | 8 bars à 20° C |
| — Température | 40° C |
| — Durée | 5 heures |

On recueille 54 g (rendement: 86%) de trifluorométhoxy-4 méthyl-4' diphényl sulfone à 96% de pureté.

On note, au cours de la réaction, une augmentation de pression due au départ de l'acide bromhydrique provenant de l'échange Br—F.

Exemple 8

On procède comme à l'exemple I avec les composés et conditions suivantes:

| | |
|---|---|
| — Acide fluorhydrique anhydre | 100 g |
| — p-chlorotrifluorométhoxybenzène | 19,7 g 0,1 mole |
| — Chlorure p-toluènesulfonyle | 19,05 g 0,1 mole |
| — Trifluorure de bore | 10 bars à 20° C |
| — Température | 30° C |
| — Durée | 22 heures |

On recueille 6,3 g (rendement: 18%)d'un mélange de trifluorométhoxy-3 chloro-6, méthyl-4' diphénylsulfone et de trifluorométhoxy-2, chloro-5, méthyl-4' diphénylsulfone brut.

Exemple 9

On procède comme à l'exemple I avec les composés et conditions suivantes:

| | |
|---|---|
| — Acide fluorhydrique anhydre | 20 g |
| — $\alpha,\alpha,\beta,\beta$-tétrafluoroéthylthiobenzène | 2 g 0,01 mole |
| — Chlorure de p-toluènesulfonyle | 1,9 g 0,01 mole |
| — Trifluorure de bore | 10 bars à 20° C |
| — Température | 20° C |
| — Durée | 8 heures |

On recueille 2,3 g (rendement: 66%) de ($\alpha,\alpha,\beta,\beta$-tétrafluoroéthylthio)-4, méthyl-4' diphénylsulfone brute.

Exemple 10

On procède comme à l'exemple 1 avec les composés et conditions suivants:

| | |
|---|---|
| — Acide fluorhydrique anhydre | 40 g |
| — $\alpha,\alpha,\beta,\beta$-pentachloroéthoxybenzène | 5 g 0,017 mole |
| — Chlorure de p-toluènesulfonyle | 3,8 g 0,02 mole |
| — Trifluorure de bore | 8 bars à 20° C |
| — Température | 30° C |
| — Durée | 5 heures |

On recueille 3,1 g (rendement: 44%) de ($\alpha,\alpha$-difluoro,$\beta,\beta,\beta$-trichloroéthoxy)-4, méthyl-4' diphénylsulfone brute.

Exemple 11

On procède comme à l'exemple 1 avec les composés et conditions suivants:

| | |
|---|---|
| — Acide fluorhydrique anhydre | 100 g |
| — $\alpha,\alpha$-difluorométhoxybenzène | 14,4 g 0,1 mole |
| — Chlorure de p-toluènesulfonyle | 19 g 0,1 mole |
| — Trifluorure de bore | 12 bars à 20° C |
| — Température | 20° C |
| — Durée | 6 heures |

On recueille 18,2 g (rendement: 61%) de $\alpha,\alpha$-difluorométhoxy-4 méthyl-4' diphénylsulfone brute.

**Revendications**

1. Procédé de préparation de $\alpha,\alpha$-difluoroalkoxy ou $\alpha,\alpha$-difluoroalkylthiophénylsulfones caractérisé en ce que l'on fait réagir un polyhalogénoalkoxybenzène ou un polyhalogénoalkylthiobenzène avec un acide sulfonique, un dérivé ou un précurseur de cet acide, en présence de trifluorure de bore en quantité telle que la pression absolue de trifluorure de bore dans l'enceinte réactionnelle soit supérieure à 1 bar et en présence d'acide fluorhydrique comme solvant.

2. Procédé selon la revendication 1 caractérisé en ce que le polyhalogénoalkoxybenzène ou le polyhalogénoalkylthiobenzène a pour formule:

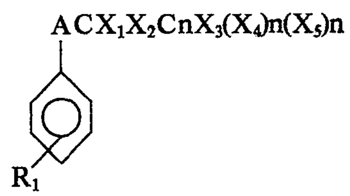

(I)

dans laquelle $X_1$ et $X_2$ identiques ou différents représentent Cl, Br, I ou F, $X_3$ $X_4$ et $X_5$ identiques ou différents représentent H, Cl, Br, I ou F, n est un nombre entier supérieur ou égal à 0 et inférieur ou égal à 5, A représente O ou S et $R_1$ représente au moins un élément choisi parmi le groupe comprenant l'hydrogène et les radicaux alkyle et alkoxy ayant de 1 à 6 atomes de carbone environ, les radicaux phényle et phénoxy substitués par au moins un groupement plus désactivant que le groupement $ACX_1X_2CnX_3(X_4)n(X_5)n$, OH,Cl, Br, I et F.

3. Procédé selon la revendication 2 caractérisé en ce que, dans la formule I, $n=0$ ou 1 et $X_1$, $X_2$ et $X_3$ sont identiques.

4. Procédé selon la revendication 3 caractérisé en ce que $X_1$, $X_2$ et $X_3$ représentent le fluor.

5. Procédé selon la l'une quelconque des revendications précédentes caractérisé en ce que l'acide sulfonique, le précurseur ou le dérivé de cet acide a pour formule générale:

$$R_2-SO_2X_6 \qquad (II)$$

dans laquelle:

$R_2$ représente un radial aliphatique ou aromatique
$X_6$ représente un halogène, OH, $OR_3$, $NH_2$, $NHR_4$, $NR_5R_6$ où
$R_3$, $R_4$ $R_5$ et $R_6$ sont un radical aromatique ou aliphatique.

6. Procédé selon la revendication 5 caractérisé en ce que, dans la formule II, $R_2$ représente un radical alkyle, phényle, alkylphényle, phénylalkyle ou phényle comportant au moins un substituant halogène, $NO_2$, CN, $NH_2$, COOH.

7. Procédé selon l'une quelconque des revendications précédentes caractérisé en ce que on utilise une quantité d'acide fluorhydrique telle que le rapport molaire de l'acide fluorhydrique au composé de formule I est compris entre 5 et 50.

8. Procédé selon l'une quelconque des revendications précédentes caractérisé en ce que l'acide fluorhydrique mis en œuvre est de l'acide fluorhydrique anhydre.

9. Procédé selon l'une quelconque des revendications précédentes caractérisé en ce que les composés de formule I et II sont utilisés en quantité sensiblement équimolaire.

10. Procédé selon l'une quelconque des revendications précédentes caractérisé en ce que on utilise une quantité de trifluorure de bore telle que la pression absolue de $BF_3$ dans l'enceinte réactionelle soit comprise entre 6 et 20 bars.

11. Procédé selon l'une quelconque des revendications précédentes caractérisé en ce que on opère

à une température comprise entre —20° C et 150° C.

**Patentansprüche**

1. Verfahren zur Herstellung von α,α-Difluoralkoxy oder α,α-Difluoralkylthiophenylsulfonen, dadurch gekennzeichnet, daß man ein Polyhalogenalkoxybenzol oder ein Polyhalogenalkylthiobenzol mit einer Sulfonsäure, einem Derivat oder einem Vorläufer dieser Säure in Gegenwart von Bortrifluorid in einer solchen Menge, daß der absolute Druck des Bortrifluorids im Reaktionsraum über 1 bar liegt, und in Gegenwart von Fluorwasserstoffsäure als Lösungsmittel umsetzt.

2. Verfahren nach Anspruch 1, dadurch gekennzeichnet, daß das Polyhalogenalkoxybenzol oder das Polyhalogenalkylthiobenzol die folgende Formel hat:

$ACX_1X_2CnX_3(X_4)n(X_5)n$

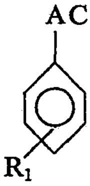

$R_1$

(I)

in der $X_1$ und $X_2$, die gleich oder verschieden sind, Chlor, Br, I oder F bedeuten, $X_3$, $X_4$ und $X_5$, gleich oder verschieden, H, Cl, Br, I oder F bedeuten können, n eine ganze Zahl größer oder gleich 0 und kleiner oder gleich 5 ist, A O oder S bedeutet und $R_1$ wenigstens ein Substituent aus der Gruppe von Wasserstoff, den Alkyl- und Alkoxyresten mit etwa 1 bis 6 Kohlenstoffatomen und den mit wenigstens einer stärker desaktivierenden Gruppe als die Gruppierung $ACX_1X_2CnX_3(X_4)n(X_5)n$, OH, Cl, Br, I und F substituierten Phenyl- und Phenoxyresten ist.

3. Verfahren nach Anspruch 2, dadurch gekennzeichnet, daß in Formel I, n gleich 0 oder 1 ist und $X_1$, $X_2$ und $X_3$ identisch sind.

4. Verfahren nach Anspruch 3, dadurch gekennzeichnet, daß $X_1$, $X_2$ und $X_3$ Fluor bedeuten.

5. Verfahren nach einem der vorangehenden Ansprüche, dadurch gekennzeichnet, daß die Sulfonsäure, der Vorläufer oder das Derivat dieser Säure die allgemeine Formel hat

$$R_2—SO_2X_6 \qquad (II)$$

in der

$R_2$ einen aliphatischen oder aromatischen Rest
$X_6$ ein Halogen, OH, $OR_3$, $NH_2$, $NHR_4$, $NR_5R_6$ bedeuten, wobei
$R_3$, $R_4$, $R_5$ und $R_6$ einen aromatischen oder aliphatischen Rest darstellen.

6. Verfahren nach Anspruch 5, dadurch gekennzeichnet, daß in Formel II $R_2$ ein Alkyl-, Phenyl-Alkylphenyl-, Phenylalkyl- oder ein Phenylrest mit wenigstens einem Substituenten aus einem Halogen, $NO_2$, CN, $NH_2$ oder COOH ist.

7. Verfahren nach einem der vorangehenden Ansprüche, dadurch gekennzeichnet, daß man eine derartige Fluorwasserstoffsäuremenge einsetzt, daß das Molverhältnis der Fluorwasserstoffsäure zur Verbindung der Formel I zwischen 5 und 50 beträgt.

8. Verfahren nach einem der vorangehenden Ansprüche, dadurch gekennzeichnet, daß die eingesetzte Fluorwasserstoffsäure wasserfrei ist.

9. Verfahren nach einem der vorangehenden Ansprüche, dadurch gekennzeichnet, daß die Verbindungen der Formel I und II in praktisch äquimolarer Menge eingesetzt werden.

10. Verfahren nach einem der vorangehenden Ansprüche, dadurch gekennzeichnet, daß man eine derartige Menge Bortrifluorid einsetzt, daß der absolute Druck des Bortrifluorids im Reaktionsraum zwischen 6 und 20 bar beträgt.

11. Verfahren nach einem der vorangehenden Ansprüche, dadurch gekennzeichnet, daß man bei einer Temperatur zwischen —20° C und 150° C arbeitet.

**Claims**

1. Process for the preparation of α,α-difluoroalkoxy or α,α-difluoroalkyl-thiophenyl-sulphones, characterised in that a polyhaloalkoxybenzene or a polyhaloalkylthiobenzene is reacted with a sulphonic acid or a derivative or a precursor of this acid, in the presence of boron trifluoride in such amount that the absolute pressure of boron trifluoride in the reaction chamber is greater than 1 bar and in the presence of hydrofluoric acid as the solvent.

2. Process according to Claim 1, characterised in that, the polyhaloalkoxybenzene or polyhaloalkylthiobenzene has the formula

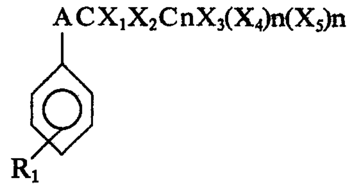

(I)

in which $X_1$ and $X_2$, which may be identical or different, represent Cl, Br, I or F, $X_3$, $X_4$ and $X_5$, which may be identical or different, represent H, Cl, Br, I or F, n is an integer greater than or equal to 0 and less than or equal to 5, A represents O or S and $R_1$ represents at least one element selected from among the group comprising hydrogen and alkyl and alkoxy radicals having from 1 to about 6 carbon atoms, phenyl and phenoxy radicals substituted by at least one group which is more highly deactivating than the group $ACX_1X_2CnX_3(X_4)n(X_5)n$, OH, Cl, Br I and F.

3. Process according to Claim 2, characterised in that, in the formula I, n = 0 or 1 and $X_1$, $X_2$ and $X_3$ are identical.

4. Process according to Claim 3, characterised in that $X_1$, $X_2$ and $X_3$ represent fluorine.

5. Process according to any one of the preceding claims, characterised in that the sulphonic acid or precursor or derivative of this acid has the general formula

$$R_2{-}SO_2X_6 \quad \text{(II)}$$

in which:

$R_2$ represents an aliphatic or aromatic radical and

$X_6$ represents a halogen, OH, $OR_3$, $NH_2$, $NHR_4$ or $NR_5R_6$ where $R_3$, $R_4$ $R_5$ and $R_6$ are an aromatic or al phatic radical.

6. Process according to Claim 5, characterised in that, in the formula II, $R_2$ represents an alkyl, phenyl, alkylphenyl, phenylalkyl or phenyl radical containing at least one halogen, $NO_2$, CN, $NH_2$ or COOH substituent.

7. Process according to any one of the preceding claims, characterised in that the amount of hydrofluoric acid used is such that the molar ratio of hydrofluoric acid to the compound of the formula I is between 5 and 50.

8. Process according to any one of the preceding claims, characterised in that the hydrofluoric acid empolyed is anhydrous hydrofluoric acid.

9. Process according to any one of the preceding claims, characterised in that the compounds of the formula I and II are used in substantially equimolar amounts.

10. Process according to any one of the preceding claims, characterised in that the amount of boron trifluoride used is such that the absolute pressure of $BF_3$ in the reaction chamber is between 6 and 20 bars.

11. Process according to any one of the preceding claims, characterised in that it is carried out at a temperature of between —20° C and 150° C.